# EUROPEAN PATENT SPECIFICATION

(11) **EP 0 542 689 B1**
(45) Date of publication and mention of the grant of the patent: **23.09.1998**
(21) Application number: 92810863.8
(22) Date of filing: 09.11.1992
(51) Int. Cl.: A61K 31/495, C07D 241/44

(54) **Use of 1-(aminoalkyl)-3-quinoxaline-2-on derivatives for the preparation of neuroprotective compositions**
Verwendung von 1-(aminoalkyl)-3-Quinoxalin-2-on Derivaten zur Herstellung von neuroprotektiven Mitteln
Utilisation de dérivés de 1-(aminoalkyl)-3-quinoxaline-2-one pour la préparation d'agents neuroprotecteurs

(30) Priority: 15.11.1991 EP 91119501
(43) Date of publication of application: 19.05.1993
(73) Proprietor: PHAFAG AKTIENGESELLSCHAFT, 9494 Schaan (LI)
(72) Inventor: Ehrenberger, Klaus, Prof. Dr., A-1090 Wien (AT); Felix, Dominik, Prof. Dr., CH-8008 Zürich (CH)
(74) Representative: Hasler, Erich, Dr.

(56) References cited:
- EP-A- 0 032 564
- WO-A-90/15606
- NEUROCHIRURGIA vol. 0, no. SU, 1981, page 332 WIDAUER J.O. ET AL 'STUDIES OF EXPERIMENTAL CEREBRAL ARTERY DILATATION (WITH CAROVERIN FUMARATE)' SUPPLEMENT, ABSTRACT NO.8.3.1
- TRENDS IN PHARMACOLOGICAL SCIENCES vol. 11, no. 9, September 1990, pages 379 - 387 MELDRUM B. ET AL 'EXCITATORY AMINO ACID NEUROTOXICITY AND NEURODEGENERATIVE DISEASE'
- BRAIN RESEARCH vol. 266, no. 1, 25 April 1983, pages 174 - 177 ISHIDA M. ET AL 'REDUCTION OF GLUTAMATE RESPONSES AT THE CRAYFISH NEUROMUSCULAR JUNCTION'
- BRITISH JOURNAL OF PHARMACOLOGY vol. 83, no. 3, November 1984, pages 813 - 830 KUDO Y. ET AL 'EFFECTS OF CAROVERINE AND DILTIAZEM ON SYNAPTIC RESPONSES, L-GLUTAMATE-INDUCED DEPOLARIZATION AND POTASSIUM EFFLUX IN THE FROG SPINAL CORD'
- IL FARMACO EDIZIONE SCIENTIFICA vol. 43, no. 7-8, August 1988, pages 613 - 618 MULE A. ET AL 'TERZ-AMMINOALCHILDERIVATI DI CHINOSSALINONI AD ATTIVITA ANALGESICA'
- PROGRESS IN NEUROBIOLOGY vol. 30, 1988, pages 399 - 435 SHINOZAKI H. 'PHARMACOLOGY OF THE GLUTAMATE RECEPTOR'

## Description

The present invention relates to the novel use of 1-(aminoalkyl)-3-(benzyl)-Quinoxaline-2-on derivatives and pharmaceutically acceptable salts thereof for the preparation of neuroprotective pharmaceutical compositions.

Molecules of the formula 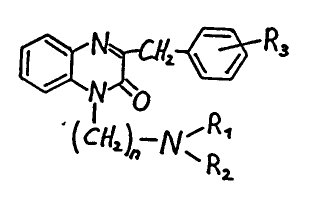 wherein
R1 and R2 are each independently hydrogen, methyl-, ethyl, propyl-, butyl, or R1 and R2 are together cycloalkyl;
R3 is methoxy, ethoxy, hydroxy, hydrogen, C1-C4 Alkyl, halogen;
n=1,2 or 3,
hereinafter referred to as 1-(aminoalkyl)-3-(benzyl)-Quinoxaline-2-on derivatives, are known since more than three decades (OE-A-228 204). 1-(aminoalkyl)-3-substituted-Quinoxaline-2-on derivatives which show in 3-position a benzyl-rest show a papaverine-like activity. (1-diethylaminoethyl-3-(p-methoxybenzyl)-1,2-dihydro-quinoxaline-2-on with the International Non-Proprietary-name (INN) Caroverine , a member of the above class, was discovered to be a powerful spasmolyticum, which is used mainly in the gastrointestinal region. The efficacy of Caroverine is attributed to its Calcium-blocking capacities whereby it blocks the Calcium mediated activation of myofibrillar ATPase, predominantly in smooth muscles.

In EP-A 032 564 the use of Caroverine-fumarate for the inhibition of the aggregation of platelets in blood, increase of the arterial circulation, treating of ischaemic heart diseases, angina pectoris etc. was described. In addition, specific Caroverine-salts had i.a. a positive effect on the cerebral blood circulation. It was also found that Caroverine is a specific Ca-antagonist (EP-A 032 564; page 18): The cardiac muscles and the smooth muscles contract under the influence of Calcium ions. The Ca-ions enter the muscle cells through specific Calcium channels and induce the contracting process from the inner cell. The Calcium channels can be opened either by local voltage changes (voltage gated ionic channels), tissue hormones or by the transmitter adrenalin. It has been found that Caroverine is capable of inhibiting 50% of the flux of Ca-ions into the cell via "voltage gated channels" opened by electrical stimulation (EP-A 032 564; page 18). The influence of Calcium on the chemical Calcium-channels has not been examined.

Widauer studied the effect of Caroverine fumarate on cerebral artery dilatation ( Neurological Surgery, suppl. to Neurochirurgia, 1981, p. 332, abstract no. 8.3.1). He, too, attributed the effect of Caroverine to its role as Ca-antagonist.

In Subsidia med. 22, 3, p.78-85 (1970) Möslinger reported positive results for a combination preparation of Caroverine and the analgeticum Novaminsulfon for treating spasms occurring after epileptic seizures. The effects of Caroverine alone for treating headache at hypertonia and for treating a cervical syndrome were mediocre in the first case and unsatisfactory in the latter case.

Recent investigations describe (Koppi et al. in Neuropsychobiology 17, p. 49-52, 1987) the use of Caroverine in treatment of acute alcohol withdrawal symptoms. Koppi et al. were inspired by Presslich and Brainin (Oesterreichische Apothekerzeitung 38/39, p. 757 (1984)) who had tested Caroverine during alcaloid withdrawal.

In contrast to the cited publications the present invention is based on the surprising discovery of 1-(aminoalkyl)-3-(benzyl)-Quinoxaline-2-on-derivatives as potent, selective and reversible glutamate receptor antagonists in the excitatory neurotransmission in the central nervous system.

Glutamate is the most abundant and important excitatory neurotransmitter in the central nervous system (Brien Meldrum in Emerging Strategies in Neuroprotection, edited by P.J.

Marangos and Harbans Lal, Birkhäuser Boston, 1992, chapter 7,). In the central nervous system Glutamate functions to promote rapid neurotransmitter depolarization by opening membrane channels chemically (in contrast to the voltage-gated inhibition of Ca++-influx in smooth muscle cells) which permit diffusion of Sodium and Calcium ions. These fast effects are mediated by different glutamate-sensitive receptor types which act as a specific "central key/lock system" and permit excitation of the subsequent nerve cell.

The glutamate receptors are extremely sensitive to a wide range of external damages (noxae), such as injuries, oxygen deficiency, metabolic disturbances, ageing processes, etc., and, under these conditions, lead to a specific kind of overstimulation of the subsequent nerve cell.

Under the conditions of an excessive stimulation of the receptors, the physiological transmitter glutamate exerts a neurotoxic action (Rothman et al. in Trends Neurosci. 10 (1987) p. 299 - 302). This excitotoxicity is mediated by the receptors N-methyl-D-aspartate (NMDA) as well as by the two nonNMDA subtypes, quisqualate and kainate (Frandsen et al., J. Neurochem. 53 (1990), pg. 297-299), whereby Ca is involved in the etiology of the glutamate-induced cell damage which can finally result in the death of the affected neurons. (Choi, Trends Neurosc., 11 (1988), p. 465-469). This glutamate receptor linked neurotoxicity has been implicated in pathological conditions like ischemia, hypoglycemia, anoxia, trauma and several severe neurodegenerative disorders (Meldrum et al., Trends pharmacol. Sci. 11 (1990), p. 379-387) as well as in neuronal death in ageing. In vitro and in vivo, glutamate receptor antagonists, as neuroprotective agents, can prevent these pathological conditions.

Therefore, there is a great interest in the development of new drugs that might selectively block the NMDA and nonNMDA receptors without influencing other receptors since such drugs could be used as neuroprotective pharmaceuticals. Applications of these pharmaceuticals could be the treatment of glutamate-induced and glutamate-receptor-mediated neurotoxic dysfunctions such as functional disturbances of the inner ear, like tinnitus and impaired hearing, and of the retina, (post)traumatic leasions, and degenerative processes of neurons in the central nervous system like Alzheimer's, Parkinson's disease etc. (Akhlaq et al., Brain Research Reviews, 16 (1991) p. 171-191)

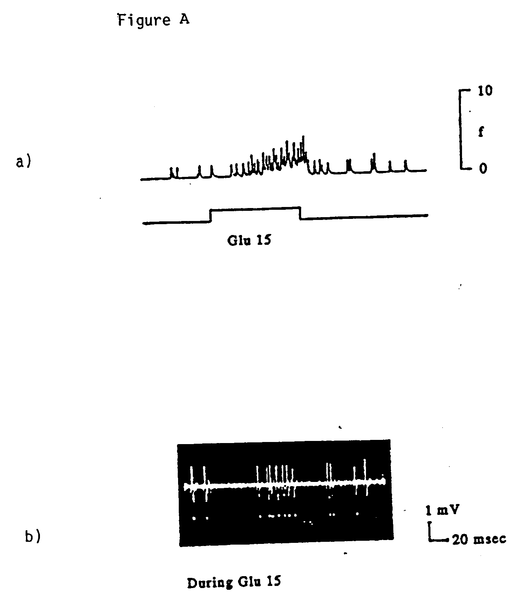

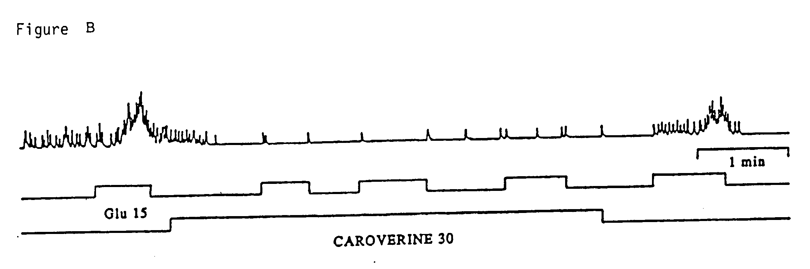

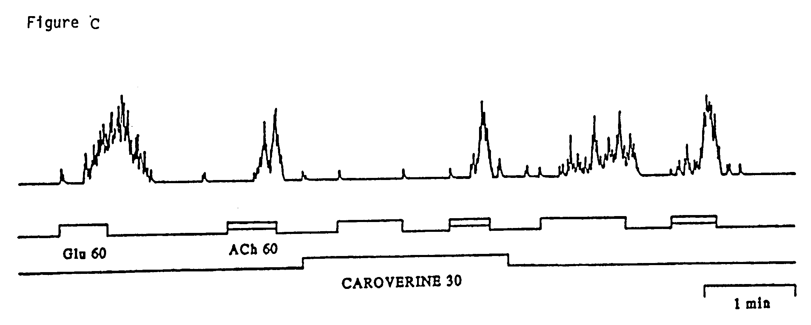

Among the presently known antagonistic substances figure 6-cyano-7-nitroquinoxaline-2,3-dione (CNQX) and 6,7-dinitroquinoxaline-2,3-dion (DNQX) (Honoré et al. in Sciene 241, p. 701-703 (1988)). DNQX and CNQX are at submicromolar concentrations competitive non-NMDA receptor antagonists binding to quisqualate receptors and at somewhat higher concentrations block the effect of NMDA receptors (Kessler et al., Brain Research 489, p. 377 -382). However, CNQX and DNQX can also act as non-competitive antagonists inhibiting the strychnine-insensitive NMDA-associated glycine recognition site meaning that important vital processes are disrupted by these compounds. Therefore a pharmaceutical use of CNQX and DNQX is not feasible.

Ishida et al. (Brain Research, 266 (1983) P. 174 - 177) studied the effect of Caroverine at the crayfish neuromuscular junction. They reasoned that Caroverine might be an open channel blocker for glutamate. Their findings, however, were rather contradictory as they did not find a Caroverine effect under more realistic, physiological conditions. Further investigations by Kudo and Shibata (Br.J. Pharmac. (1984), 83, p. 813-820) published one year later led to the opinion that Caroverine was an unspecific calcium-channel-blocking agent but not a glutamate antagonist. They also found that the effect on glutamate does not take place at its receptors.

The present invention is based upon results received by examining the effects of 1-(aminoalkyl)-3-(benzyl)-Quinoxaline-2-on-derivatives directly in the mammalian cochlea and upon subsequent clinical studies.

In the mammalian cochlea, there is strong evidence that two classes of glutamate-receptors, the N-methyl-D-aspartate (NMDA) subclass and the nonNMDA subclass, comprising the quisqualate-and kainate subtypes, mediate postsynaptic glutamatergic neurotransmission between the inner hair cells (IHC) and the peripheral dendrites of the afferent neurons (Puel et al., Hear. Res., 51, (1991), p. 255-264). Using physiological techniques, up to now, no other types of receptor channels could be found at the IHC synapses. Therefore, the IHC synapse is an elegant and easily accessible model for prominent excitatory synapses corresponding to similar structures of the central nervous system (CNS) since the physiological structures and mechanisms in the cochlea correspond to similar structures and mechanisms in the central nervous system. These findings are therefore representative for similar synaptic connections of the central nervous system.

The effectiveness of the 1-(aminoalkyl)-3-(benzyl)-Quinoxaline-2-on-derivatives as potential glutamate receptor antagonists was studied in the light of the neurotransmission between the inner hair cells (IHC) and the peripheral dendrites of the afferent neurons in the mammalian cochlea.

Experiments were performed on adult pigmented guinea pigs (300 - 700 g) of both sexes, anaesthetised with a combination of Rompun (Bayer, Leverkusen, Germany) and Innovar-Vet (Pitman-Moore, Mundelin, Ill., USA). Supplementary low doses of pentobarbital (Nembutal, Abbott, Chicago, USA) were given periodically throughout the experiments. Rectal temperature was maintained within physiological limits. After tracheotomy for artificial respiration, the auditory bulla was approached ventrolaterally and the cochlea was exposed. A small opening was drilled into the cochlea bone over the pigmented stria vascularis and the ligamentum spirale of the third turn.

Multibarrel microelectrodes with overall tip diameter of 1.5-2 µm were inserted and microdriven almost parallel to the tectorial membrane until the subsynaptic region of the IHC layer was reached at a depth of 200 - 260 µm. The occurrence of phasic activity was used to identify the location precisely, since such activity occurs in the immediate vicinity of the initial post-synaptic structures.

Recordings of extracellular action potentials were obtained using a 2M NaCl-filled barrel of the 5-barrel glass micropipette. Recordings were analysed on a rate meter (NE 4667) and displayed continuously on a UV-oscillograph (Bell&Howell 5-137). Three other channels of the micropipette contained the solutions to be ejected microiontophoretically with the appropriate anionic and cationic currents: L-glutamic acid (0.5M, pH 3.5, adjusted with HCl); Caroverine-HCl (1-diethylaminoethyl-3-(p-methoxybenzyl)-1,2-dihydro-quinoxaline-2-on, 0.05 M, natural pH = 6.0).

In order to avoid electrically induced artefacts, compensation currents were applied through an NaCl-filled channel. Correct positioning of the tip of the micropipette in the synaptic region of the IHC's resulted in a spontaneous phasic activitiy characterised by graded potentials. Glutamate, the agonist of NMDA and nonNMDA receptors, enhances the firing rate in the majority of the fibers tested (n=29 out 31).

The experimental results are now explained with reference to the drawings:
- Fig. A: shows the excitatory effect of L-glutamate on a spontaneously firing fibre in the subsynaptic region of an IHC,
a) time frequency histogram of spike-activity during L-glutamate (Glu, 15 nA) application. The length of the perisynaptic release of the agent from the micropipette is indicated by the bar below the histogram; integrating firing frequency immediately at right.
b) the same glutamate-induced irregular spike-activity characterized by bursts, illustrated on an original oscilloscope trace (calibrations: 20 msec, 1 mV)
- Fig. B: shows the effect of Caroverine on glutamate-induced spike-activity, demonstrated on time frequency histograms. The excitatory action of L-glutamate (Glu, 15 nA) is blocked by Caroverine (30 nA for about 5 min) in a potent but reversible manner,
- Fig. C: shows the neural response to a four times higher amount of L-glutamate (Glu, 60 nA) and the same amount of acetylcholine (Ach, 60 nA) before, during and after ejection of Caroverine, using the same amount as in Fig. B (Caroverine, 30 nA). Caroverine selectively blocks the glutamate action whereas the acetylcholine-induced firing rate remaines unaffected. The ejection-periods of substances ejected from the same micropipette at the same synapse are indicated by different bars.

As shown in the original tracing a) of Fig. A the glutamate-induced excitatory effect (glutamate: 15nA for 100 msec) is characterised by an irregular discharge interrupted by bursts. The time frequency reflects the diffusion time of the agent between the micropipette and the synaptic cleft.

Caroverine ejected simultaneously with glutamate (Fig. B) antagonizes the membrane response to glutamate in an enduring but reversible manner.

In a second series of experiments, the selectivity of the described Caroverine effect was tested by comparing successively the putative antagonism of Caroverine on the glutamate-induced as well as on the acetylcholine-induced postsynaptic excitation of identical dendritic fibers. As shown in Fig. C Caroverine applied iontophoretically in an adjusted dosage, which promises a nanomolar concentration of the agent on the synapse, blocked regularly and exclusively the potent glutamate-receptors, but exhibited no effect on the membrane response to the excitatory efferent transmitter substance acetylcholine.

Another effective 1-(aminoalkyl)-3-(benzyl)-Quinoxaline-2-on-derivative is 1-diethylaminoethyl-3-(p-hydroxybenzyl),-1,2-dihydro-quinoxaline-2-on or a pharmaceutically acceptable salt thereof.

Thus it is experimentally established that 1-(aminoalkyl)-3-(benzyl)-Quinoxaline-2-on-derivatives are a potent, reversible and selective class of new glutamate receptor antagonists on the excitatory afferent synapses of the cochlear inner hair cells. The application of Caroverine and its salts, respectively, also in high doses, produces no critical side-effects. As Caroverine does not impair other physiological processes of the CNS its use as neuroprotective pharmaceutical is straightforward.

The pharmaceutically effective 1-(aminoalkyl)-3-(benzyl)-Quinoxaline-2-on-derivatives may be incorporated in a conventional systemic dosage form (oral, rectal and parenteral), such as a tablet, capsule, elixier or injectable. The above dosage forms will also include the necessary carrier material, excipient, lubricant, bulking agent or the like.

The dose administered can be adjusted according to age, weight and condition of the patient. Thus for oral administration, a satisfactory result may be obtained employing the active substance e.g. Caroverine in an amount within the range of from about 0.1 mg/kg to about 5 mg/kg and preferably from about 0.5 mg/kg to about 2 mg/kg. If Caroverine-salts are used then the amounts to be administered should be adjusted according to the increase in molecular weight.

Liquid formulations can also be prepared by dissolving or suspending the active substance in a conventional liquid vehicle acceptable for pharamceutical administration.

For the parenteral administration the active substance will be employed in an amount within the range of from about 0.1 mg/kg to about 5 mg/kg and preferably from about 0.8 mg/kg to about 2 mg/kg.

The neuroprotective effect of Caroverine was tested in a clinical study with patients who suffered from tinnitus. The etiology of tinnitus can be very different. One of the most frequent tinnitus type is, however, the cochlear tinnitus. If a functional disturbance between the IHC and the peripheral dendrites of the efferent neurons is deemed to be the initiating, etiological factor, then this is called a "cochlear synaptic tinnitus". A neuroprotective pharmaceutical like Caroverine should therefore have a positive effect on the cochlear synaptic tinnitus.

In a pilot study Caroverine was applied to 72 patients suffering from tinnitus. Caroverine was applied intravenously (160 mg/100 ml physiological NaCl-solution). The real dosage depended on the tinnitus reduction perceived by the individual patient and varied between 70 and 160 mg.

The effectiveness of Caroverine was measured by a subjective rating and tinnitus-matching before and after the infusion of the drug. 66.7 % of the patients noted a tinnitus reduction of at least 50% in absolute values immediately after infusion of Caroverine. No sincere side-effects could be observed.

## Claims

1. Use of compounds of the formula 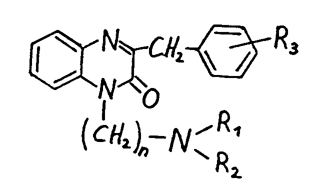 wherein
R1 and R2 are each independently hydrogen, methyl-, ethyl, propyl-, butyl, or R1 and R2 are together cycloalkyl;
R3 is methoxy, ethoxy, hydroxy, hydrogen, C1-C4 Alkyl, halogen;
n=1,2 or 3
or pharmaceutically acceptable salts thereof for the preparation of neuroprotective compositions for the prevention or treatment of neurotoxicity and functional disturbances of the central nervous system with the exception of epileptic seizures and alcohol withdrawal symptoms.

2. Use according to claim 1 wherein R1 and R2 are ethyl, n=2 and R3 is methoxy so that the molecule is 1-diethylaminoethyl-3-(p-methoxybenzyl)-1,2-dihydro-quinoxaline-2-on (INN:Caroverine) or a pharmaceutically acceptable salt thereof.

3. Use according to claim 1 wherein R1 and R2 are ethyl, n=2 and R3 is hydroxy so that the molecule is 1-diethylaminoethyl-3-(p-hydroxybenzyl)-1,2-dihydro-quinoxaline-2-on or a pharmaceutically acceptable salt thereof.

4. Use according to one of the claims 1 to 3 for the prevention or treatment of glutamate-induced and glutamate receptor mediated neurotoxicity and functional disturbances of the central nervous system

5. Use according to one of the claims 1 to 4 for treating functional disturbances in the inner ear, such as post-synaptic tinnitus and impaired hearing.

6. Use according to one of the claims 1 to 4 for treating functional disturbances of the retina accompanied by loss of vision.

7. Use according to one of the claims 1 to 4 for treating (post)traumatic cerebral leasions, such us concussion, and cerebral damages following oxygen deficiency (asphyxation, drowning).

8. Use according to one of the claims 1 to 4 for treating functional disturbances and degenerative processes of neurons in the central nervous system such as e.g. Alzheimer's, Huntington's and Parkinsons's disease and Wernicke/Korsakoff and Jakob/Creutzfeld syndrome.

## Patentansprüche

1. Verwendung von Verbindungen der Formel 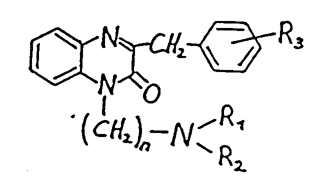 wobei
R1 und R2 unabhängig voneinander Wasserstoff, Methyl-, Ethyl, Propyl-, Butyl, oder R1 und R2 zusammen ein Cycloalkyl sind;
R3 ist Methoxy, Ethoxy, Hydroxyl, Wasserstoff, C1-C4 Alkyl, Halogen;
n=1,2 oder 3
oder eines pharmazeutisch unbedenklichen Salzes derselben für die Herstellung von neuroprotektiven Zusammensetzungen zur Verhinderung oder Behandlung von Neurotoxizität und funktionellen Störungen des zentralen Nervensystems mit der Ausnahme von epileptischen Anfällen und Alkoholentzugserscheinungen.

2. Verwendung gemäss Anspruch 1, wobei R1 und R2 Ethyl sind, n=2 und R3 ist Methoxy, sodass das Molekül 1-diethylaminoethyl-3-(p-methoxybenzyl)-1,2-dihydro-Chinoxalin-2-on ist (INN:Caroverin) oder ein pharmazeutisch unbedenkliches Salz davon ist.

3. Verwendung gemäss Anspruch 1, wobei R1 und R2 Ethyl sind, n=2 und R3 ist Hydroxy, sodass das Molekül 1-diethylaminoethyl-3-(p-hydroxybenzyl)-1,2-dihydro-Chinoxalin-2-on oder ein pharmazeutisch unbedenkliches Salz davon ist.

4. Verwendung gemäss einem der Ansprüche 1 bis 3 zur Verhinderung oder Behandlung von Glutamat-induzierter und Glutamat-Rezeptor vermittelter Neurotoxizität und funktionellen Störungen des zentralen Nervensystems.

5. Verwendung gemäss einem der Ansprüche 1 bis 4 zur Behandlung von funktionellen Störungen des Innenohres, wie des post-synaptischen Tinnitus und Gehörverlustes.

6. Verwendung gemäss einem der Ansprüche 1 bis 4 zur Behandlung von funktionellen Störungen der Retina verbunden mit einem Sehverlust.

7. Verwendung gemäss einem der Ansprüche 1 bis 4 zur Behandlung von(post) traumatischen cerebralen Verletzungen, wie Hirnerschütterung und cerebralen Schädigungen als Folge eines Sauerstoffmangels. (Asphyxation, Ertrinken).

8. Verwendung gemäss einem der Ansprüche 1 bis 4 zur Behandlung von funktionellen Störungen und degenerativen Prozessen von Neuronen im zentralen Nervensystem wie z.B. der Alzheimer-, Huntington's und Parkinsonscher Erkrankung und des Wernicke/Korsakoff und Jakob/Creutzfeld Syndrom.

## Revendications

1. Utilisation de composés de formule 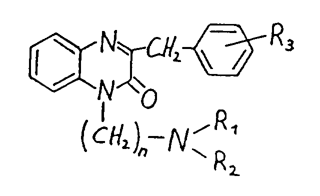 dans laquelle
R₁ et R₂ sont chacun indépendamment un atome d'hydrogène, un groupe méthyle, éthyle, propyle, butyle, ou R₁ et R₂ sont ensemble un groupe cycloalkyle ;
R₃ est un groupe méthoxy, éthoxy, hydroxy, un atome d'hydrogène, un groupe alkyle en C₁ à C₄, un atome d'halogène ;
n = 1, 2 ou 3,
ou de sels pharmaceutiquement acceptables de ceux-ci, pour la préparation de compositions neuroprotectrices pour la prévention ou le traitement d'une neurotoxicité et de perturbations fonctionnelles du système nerveux central, à l'exception des crises d'épilepsie et des symptômes du sevrage de l'alcool.

2. Utilisation selon la revendication 1, dans laquelle R₁ et R₂ sont un groupe éthyle, n=2 et R₃ est un groupe méthoxy, de sorte que la molécule est la 1-diéthylaminoéthyl-3-(p-méthoxybenzyl)-1,2-dihydroquinoxaline-2-one (dénomination commune internationale : Caroverine) ou un sel pharmaceutiquement acceptable de celle-ci.

3. Utilisation selon la revendication 1, dans laquelle R₁ et R₂ sont un groupe éthyle, n=2 et R₃ est un groupe hydroxy, de sorte que la molécule est la 1-diéthylaminoéthyl-3-(p-hydroxybenzyl)-1,2-dihydroquinoxaline-2-one ou un sel pharmaceutiquement acceptable de celle-ci.

4. Utilisation selon l'une quelconque des revendications 1 à 3, pour la prévention ou le traitement d'une neurotoxicité et de perturbations fonctionnelles du système nerveux central dues au glutamate ou aux récepteurs de glutamate.

5. Utilisation selon l'une quelconque des revendications 1 à 4 pour traiter des perturbations fonctionnelles de l'oreille interne, comme un bourdonnement d'oreille post-synaptique et une déficience auditive.

6. Utilisation selon l'une quelconque des revendications 1 à 4 pour traiter des perturbations fonctionnelles de la rétine accompagnées par une perte de vision.

7. Utilisation selon l'une quelconque des revendications 1 à 4 pour traiter des lésions cérébrales (post)traumatiques, comme une commotion, et des dommages cérébraux faisant suite à une déficience d'oxygène (asphyxie, noyade).

8. Utilisation selon l'une quelconque des revendications 1 à 4 pour traiter des perturbations fonctionnelles et des processus dégénératifs des neurones dans le système nerveux central tels que par exemple la maladie d'Alzheimer, de Huntington et de Parkinson et le syndrome de Wernicke/Korsakoff et de Creutzfeld/Jakob.
